# EUROPEAN PATENT APPLICATION

(11) **EP 1 655 040 A1**
(43) Date of publication of application: **10.05.2006**
(21) Application number: 04024102.8
(22) Date of filing: 08.10.2004
(51) Int. Cl.: A61K 49/22, A61K 49/00

(54) **Contrast agent formulations for the visualization of the lymphatic system**

(71) Applicant: Bracco Imaging, S.P.A., 20134 Milano (IT)
(72) Inventor: de Haen, Christoph, 20134 Milano (IT)
(74) Representative: Poletti, Marco

(57) **Abstract**

The present invention relates to the field of diagnostic imaging and provides compositions of ultrasound contrast agents, particularly gas-filled microvesicles suspensions, in combination with vital dyes.

The compositions of the invention are advantageously used in the visualization of the lymphatic system, particularly for the detection of the sentinel lymph node or nodes of a tumor.

## Description

The present invention relates to the field of diagnostic imaging and, more particularly, it relates to formulations of ultrasound contrast agents in combination with visible marking agents, in particular vital dyes.

The formulations of the invention may be used in a variety of diagnostic imaging situations including ultrasound echographic and intraoperative optical visualization of the lymphatic system and, more particularly, of the sentinel lymph node or nodes of a tumor.

### Background of the invention

Development of ultrasound techniques and diagnostic applications thereof has taken advantage, in recent years, from a variety of formulations of contrast agents useful in the imaging of blood and lymph vessels, organs and tissues, of human or animal bodies.

Most of the above formulations are primarily designed for intravenous or intra-arterial administration in conjunction with the use of medical echographic equipment.

A first class of injectable formulations of ultrasound contrast agents include, for instance, suspensions of gas bubbles having a proper diameter of a few microns dispersed in aqueous media.

Of particular interest, within this class, are gas bubbles which are stabilized by means of suitable additives or ingredients such as, for instance, fatty acids and phospholipids, or by entrapping or encapsulating the gas or a precursor thereof in a variety of systems. These stabilized gas bubbles are widely known in the art and are generally referred to as "microvesicles".

Microvesicles, in their turn, may be conveniently divided into two main categories.

A first category of stabilized bubbles or microvesicles is generally referred to as "microbubbles". Microbubbles comprise aqueous suspensions in which the bubbles of gas are bound at the gas/liquid interface by a very thin envelope (film) involving a stabilizing amphiphilic material disposed at the gas to liquid interface.

A few methods are known in the art for preparing the microbubble suspension. Typically, the microbubble suspension is prepared by suspending, in a suitable aqueous solution for injection, a suitably prepared solid formulation containing the amphiphilic material, e.g. freeze-dried preformed liposomes or freeze-dried or spray-dried phospholipid solutions, kept under an atmosphere of a suitable gas, for instance air or nitrogen or perfluorinated gases or mixtures thereof. The microbubble suspension thus generated can be administered, preferably shortly after its preparation.

In a second way, the microbubble suspension is prepared from a suspension of gas-free particles containing the amphiphilic material kept under the aforementioned gas atmosphere which, under vigorous agitation, forms a suspension of microbubbles.

In a third way, the microbubble suspension is prepared by suspending, in a suitable aqueous solution for injection, a suitably prepared solid formulation containing the amphiphilic material together with materials that produce a gas upon contact with water, or that liberate a gas from the suspension medium or the blood plasma or both, once injected.

Non limiting examples of aqueous suspensions of the above gas microbubbles and preparations thereof are disclosed, for instance, in US 5,271,928, US 5,445,813, US 5,413,774, US 5,556,610, US 5,597,549, US 5,827,504, WO 97/29783, WO 04/069284, EP 0855186, US 5,141,738, US 6,306,366, EP 0365467, all of which are herewith incorporated by reference in their entirety.

A second category of injectable formulations of ultrasound contrast agents comprises formulations containing microvesicles of the kind generally referred to as "microballoons" or "microcapsules" and includes suspensions in which the gas bubbles are surrounded by a solid material envelope of a lipid or of natural or synthetic polymers.

Non limiting examples of microballoons and preparations thereof are disclosed, for instance, in US 5,711,933, US 6,333,021, US 5,611,344, US 6,045,777, US 6,132,699 and WO O1/12071, all of which herein incorporated by reference in their entirety.

Ultrasound contrast agents, hence including the above microbubbles and microballons, are currently used in a variety of diagnostic applications for the visual imaging of organs and tissues through known echographic instrumentations and techniques. Among the said diagnostic application is, for instance, the imaging of the lymphatic system.

The lymphatic system comprises vessels or ducts that begin in tissues and are designed to collect interstitial fluid and to carry it as lymph to local lymph nodes. The lymph is therein filtered and processed and sent to the next of a series of consecutive lymph nodes down the line, until it enters the blood.

During filtration the macrophages inside the nodes remove, via phagocytosis, particulate and cell material from the lymph. Large particles such as cells may become trapped in the small lymphatic passages in the nodes. When cancer occurs in tissues or organs, dislodging of cancerous cells into the lymphatic system may occur as well. These cancerous cells may thus become entrapped in the lymph node where they adhere to tissue and grow, forming metastatic foci.

However, whilst in early stages of cancer development in the node the cancer remains limited to the node itself, the nodal deposit can also subsequently grow to totally replace the node and/or can spread downstream to the next node. The lymph nodes that drain the tissue or organ of interest, like the cancerous tissue, are the so-called regional lymph nodes and the first node that traps the cancer-derived metastatic cells is referred to as the sentinel lymph node (see, for a general reference, WO 04/030651). In rare cases the tumor lymph drains into more than one drainage basin, leading to multiple sentinel lymph nodes.

In cancer surgery, known techniques like lymphadenectomies are often performed with the aim of eliminating cancer disease which has spread to lymph nodes. The intervention may be made by open surgery or by endoscopically guided surgery. Typically, the surgeon removes the fat tissue strings which, from experience, are known to include the lymph nodes and which are located close to the large blood vessels, and above all the veins.

The outcome of this type of surgery may vary to a great extent as it may depend, essentially, upon the identification of the single lymph nodes in the fat masses, all having similar colour and consistency (see, for a reference, WO 00/35490). In the light of the above limitations, the need of easily detecting and inspecting the lymphatic system and, more particularly, the sentinel lymph node, is of utmost importance in the early stage of cancer diagnosis and therapy.

Certain lymphatic pathways may be visualized by various imaging techniques after injection of appropriate contrast agents directly into lymphatic vessels (direct lymphography). This approach is applicable to X-ray imaging, magnetic resonance imaging, echographic imaging and nuclear imaging. Direct lymphography is a skill-requiring open surgery procedure under anesthesia. It is only practical when large lymphatic vessels are accessible. For example, after administration of X-ray contrast agents into lymphatic vessels of the foot, a few leg lymph nodes and pelvic and abdominal lymph nodes may be thus visualized. However, direct lymphography is not usually suitable for the identification of the sentinel lymph node(s) of a tumor.

Several means specifically intended for the identification of the sentinel lymph node(s) and based on the use of contrast agents are known in the art. They may involve indirect lymphography, i.e. the imaging of the lymphatic system after injection of contrast agents outside the lymphatic system, e.g. interstitially, subcutaneously, subdermally, intradermally, subareolarly, periareolarly, intraperitoneally or intravenously. Contrast agents injected into tissue interstitium in general, e.g. into peritumoral regions in particular, do reach the lymphatic system but to degrees depending on their nature. In this respect, as truly soluble agents with molecular diameters substantially below 3 nm tend to enter the blood vasculature just as easily as the lymphatic system, they do not offer a good lymphatic contrast.

Commonly available soluble contrast agents are much smaller than the above limit and, hence, do not get trapped in lymph nodes, yet do get into the vasculature. As a result, their concentration in the nodes does not reach sufficient levels to permit good visualization.

Particulate contrast agents, instead, enter the lymphatic system preferentially over blood vessels, provided they contain particulates of a suitable size range, typically with diameters between 3 and 900 nm.

Particles having a diameter up to about 900 nm have been found to enter lymphatic systems from the interstitium, whereas particles substantially larger are reported not to do so (see Wolf G. et al.; Percutaneous lymphography using particulate fluorocarbon emulsions; US 5,114,703).

As an example, WO 01/12071 describes an ultrasound contrast agent composition with diameters in the range of about 100 to 800 nm for sentinel lymph node detection after subcutaneous administration.

Suitably sized particulate contrast agents injected interstitially allow imaging of the lymph nodes, in part because after opsonization they get trapped by macrophages in the nodes; passage from interstitium to the lymphatic system is reported to be accelerated by massaging the interested area.

Subcutaneous and subdermal injection of contrast agents has requirements and limitations for successful imaging of the lymphatic system very similar to the interstitial injection. The process of diffusion of the contrast agent, in fact, is rather slow and requires as well the massaging at the injection site.

Among the currently used methods for the identification of the sentinel lymph nodes is lymphoscintigraphy that, despite the above limitations, is today mostly performed by subcutaneous or interstitial injection. More recently in the case of breast subareolar and periareolar injection is being proposed.

The scintigraphic contrast agents used are colloidal contrast agents prepared from commercial products by further filtration in the hospitals. No scintigraphic contrast agent, at present, is registered with the indication for sentinel lymph node detection.

In the case of breast tumor scintigraphic sentinel lymph node detection involves the injection, under local or general anesthesia, of a scintigraphic contrast agent, usually administered several hours before the start of the diagnostic imaging. Typically, the biopsy of the identified sentinel lymph node(s) can be performed the subsequent day only.

As an additional drawback, scintigraphy requires to be performed within a Nuclear Medicine Department with heavy and immobile imaging equipment, a nuclear pharmacy and authorized personnel which, in many hospitals, are not available. For biopsy of the sentinel lymph node(s) the patient usually has to be transferred to the surgical department. The whole procedure is thus lengthy and involves, at least, two distinct hospital departments.

Another known method for detecting sentinel lymph nodes involves the injection of a blue vital dye.

The visual identification of the sentinel lymph node(s) is then performed intraoperatively, that is by observing the dye at the region suspected to contain the sentinel lymph node. In particular, when intraoperative lymphatic mapping is performed with a blue dye, the sentinel lymph node may be identified by following a blue-stained lymphatic channel draining from the primary tumor site to a blue stained node, the sentinel lymph node (see, for a reference, Donald L. Morton et al.; Ann. Surg. Oncol., Vol. 6, No. 1, 1999).

Moreover, the blue dye allows the labeling of all of the lymph nodes and the sentinel one needs to be identified through the earliest time of arrival of the dye. So far, given the size of the area to be kept under observation together with the fact that the dye tends to spread around all of the lymphatic structures, this visualization is rather troublesome. Clearly, this method may suffer from excessive surgical exploration.

Combined techniques have been also developed which may comprise, for instance, preoperative lymphoscintigraphy by using Tc-99m labeled sulfur colloid followed by blue dye administration.

Nevertheless, whilst the blue dye is injected 5-15 min before intraoperative lymphatic mapping and sentinel lymph node detection, additional injections of labeled sulfur colloid may be required if more than 24 h has passed since preoperative lymphoscintigraphy (see Donald L. Morton et al.).

US 6,205,352 describes the detection of the sentinel lymph node(s) of a tumor by injection of a non-radioactive contrast agent followed by imaging with a modality capable of detecting the contrast agent. Among the contrast agents and imaging modalities, ultrasound contrast agents and echographic detection methods are also claimed therein.

In addition, it is therein reported that such agents "are preferably about 10 to about 200 nanometers in diameter". According to this latter aspect, however, it is known to the skilled man that with those sizes ultrasound contrast agents have essentially no echographic efficacy.

It is also known in the art that flexible microbubbles injected interstitially, subcutaneously or intradermally can reach the lymphatic system even if they are larger than 900 nm in diameter. Probably these products can deform on their own or under the action of massage, thereby assuming adaptive shapes for entering the lymphatic system.

US 6,444,192 describes the echographic imaging of the lymphatic ducts and nodes after the administration, into an appropriate site other than blood or lymph structures, of a particulate ultrasound contrast agent.

From all of the above there is the need, in the field of diagnostics, of a technique for the imaging of the lymphatic system that allows an easy, rapid, reproducible and robust detection of the sentinel lymph node(s).

### Description of the invention

We have now found that visualization of the lymphatic system, in particular identification of the sentinel lymph node or nodes of a tumor, can be advantageously achieved by combining ultrasound imaging techniques with vital dyes techniques.

We have thus found, unexpectedly, that ultrasound contrast agents may be conveniently formulated in combination with vital dyes and thus administered, preferably shortly before diagnostic imaging.

It is therefore a first object of the present invention a composition comprising an ultrasound contrast agent in combination with a vital dye.

Once administered to a subject undergoing diagnostic imaging, for instance of the lymphatic system, the composition of the invention allows optimal visualization and inspection of the sentinel lymph node and minimizes surgical exploration during sentinel lymph node excision.

Very advantageously, in fact, the use of the composition of the invention enables the surgeon to locate the sentinel lymph node by ultrasound imaging and, hence, the optimal site of operative incision just prior to surgery, whilst allowing visual recognition of the lymphatic structures and the lymph nodes before their optional excision.

From the above, it is clear to the skilled man that in the composition of the invention, a diagnostically effective amount of both the components is required.

With the terms "diagnostically effective amount" we intend, unless otherwise provided, any amount of both the ultrasound contrast agent and the dye which is sufficient to enable echographic imaging and visual imaging, respectively.

Typically, the amounts of the ultrasound contrast agent and the vital dye substantially correspond to the same amount that would have been administered by using each of them separately, according to known techniques.

Preferably, the composition of the invention is administered so as to provide amounts of the ultrasound contrast agent ranging from about 0.01 µl to about 6.0 µl of gas, inside microvesicles, per patient.

Likewise, through the administration of the composition of the invention, amounts of vital dye ranging from about 1 µmol to about 100 µmol may be thus administered, in one or multiple sites.

In the present description, unless otherwise indicated, with the term vital dye we intend any known dye being developed as suitable for the parenteral administration to human and animal bodies.

Preferably, with the aim of providing optimal ocular visibility, the dye is characterized by having at least one light absorption maximum at wavelengths comprised between 500 nm and 900 nm and, even more preferably, between 520 and 750 nm.

Suitable vital dyes according to the invention may include, for instance, water soluble dyes selected among the classes of triphenylmethane dyes, cyanine dyes, mero-cyanine dyes, styryl dyes, thiazine dyes, diazo dyes, porphyrins and their ring-enlarged higher homologues and phthalocyanins and their ring-enlarged higher homologues dyes.

Additional vital dyes according to the invention may also comprise suitable complexes with metals, e.g. gadolinium, copper, cobalt and magnesium, so as to give rise, for instance, to metalloporphyrins, metallochlorins, metallophthalocyanins and higher homologues thereof.

Non limiting examples of vital dyes according to the invention may thus include, for instance, Evans blue [CAS 61-73-4], patent blue V [CAS 3536-49-0], patent blue VF [CAS 129-17-9], isosulfan blue [also known as Patent blue violet, CAS 68238-36-8], indocyanine green monosodium salt [CAS 3599-32-4], methylene blue [CAS 314-13-6], sulfobromophthaleine [123359-42-2], copper phthalocyanine tetrasulfonate [27360-85-6], gadolinium texaphyrin [156436-89-4]. According to a preferred embodiment of the invention, the vital dye is selected between Evans blue and patent blue VF.

Vital dyes are commercially available compounds or may be easily obtained by well known chemical syntheses. Commercial products may be further purified by classical means such as dissolution, filtration and reprecipitation as well as by chromatography. In particular, available solutions of these products may be purified from contaminant salts by electrodialysis and from particulates and endotoxins by ultrafiltration. Thus, they may be purified until they reach the required degree of purity for parenteral administration to mammals, including humans.

In the present description, unless otherwise specified, with the term ultrasound contrast agent we intend any contrast agent known to be used for echographic diagnostic imaging techniques.

Preferably, said ultrasound contrast agents are those previously reported and shortly referred to as gas-containing or gas-filled microbubble or microballoon suspensions.

For details about the gas-filled microvesicles, their main components and optional excipients, the gases themselves, their appearance as well as the method for their preparation, see the following extensive section.

### Ultrasound contrast agents: gas-filled microvesicles

The gas-filled microbubbles are those known in the art and comprise bubbles of gas dispersed in an aqueous medium which are stabilized by a thin envelope comprising an amphiphilic compound disposed at the gas to liquid interface. Said stabilizing envelope, sometimes referred to as an "evanescent envelope", has in general a thickness of less than 5 nm, typically of about 2-3 nm, thus often amounting to a substantially monomolecular layer.

The amphiphilic compound included in the microbubbles' envelope can be a synthetic or naturally-occurring biocompatible compound and may include, for example a film forming lipid, in particular a phospholipid. Examples of amphiphilic compounds include, for instance, phospholipids; lysophospholipids; fatty acids, such as palmitic acid, stearic acid, arachidonic acid or oleic acid; lipids bearing polymers, such as chitin, hyaluronic acid, polyvinylpyrrolidone or polyethylene glycol (PEG), also referred as "pegylated lipids"; lipids bearing sulfonated mono- di-, oligo- or polysaccharides; cholesterol, cholesterol sulfate or cholesterol hemisuccinate; tocopherol hemisuccinate; lipids with ether or ester-linked fatty acids; polymerized lipids; diacetyl phosphate; dicetyl phosphate; ceramides; polyoxyethylene fatty acid esters (such as polyoxyethylene fatty acid stearates), polyoxyethylene fatty alcohols, polyoxyethylene fatty alcohol ethers, polyoxyethylated sorbitan fatty acid esters, glycerol polyethylene glycol ricinoleate, ethoxylated soybean sterols, ethoxylated castor oil or ethylene oxide (EO) and propylene oxide (PO) block copolymers; sterol aliphatic acid esters including, cholesterol butyrate, cholesterol iso-butyrate, cholesterol palmitate, cholesterol stearate, lanosterol acetate, ergosterol palmitate, or phytosterol n-butyrate; sterol esters of sugar acids including cholesterol glucuronides, lanosterol glucoronides, 7-dehydrocholesterol glucoronide, ergosterol glucoronide, cholesterol gluconate, lanosterol gluconate, or ergosterol gluconate; esters of sugar acids and alcohols including lauryl glucoronide, stearoyl glucoronide, myristoyl glucoronide, lauryl gluconate, myristoyl gluconate, or stearoyl gluconate; esters of sugars with aliphatic acids including sucrose laurate, fructose laurate, sucrose palmitate, sucrose stearate, glucuronic acid, gluconic acid or polyuronic acid; saponins including sarsasapogenin, smilagenin, hederagenin, oleanolic acid, or digitoxigenin; glycerol or glycerol esters including glycerol tripalmitate, glycerol distearate, glycerol tristearate, glycerol dimyristate, glycerol trimyristate, glycerol dilaurate, glycerol trilaurate, glycerol dipalmitate,; long chain alcohols including n-decyl alcohol, lauryl alcohol, myristyl alcohol, cetyl alcohol, or n-octadecyl alcohol; 6-(5-cholesten-3β-yloxy)-1 -thio-β-D-galactopyranoside; digalactosyl-diglyceride; 6-(5-cholesten-3β-yloxy)hexyl-6-amino-6-deoxy-1-thio-β-D-galactopyranoside; 6-(5-cholesten-3β-yloxy)hexyl-6-amino-6-deoxyl-1-thio-β-D-manno-pyranoside; 12-(((7'-diethylaminocoumarin-3-yl)carbonyl)methylamino)octadecanoic acid; N-[12-(((7'-diethylaminocoumarin-3-yl)carbonyl)methylamino)octadecanoyl]-2-aminopalmitic acid; N-succinyldioleylphosphatidylethanolamine; 1,2-dioleyl-sn-glycerol; 1,2-dipalmitoyl-sn-3-succinylglycerol; 1,3-dipalmitoyl-2-succinylglycerol; 1-hexadecyl-2-palmitoylglycerophosphoethanolamine or palmitoylhomocysteine; alkylamines or alkylammonium salts, comprising at least one (C₁₀-C₂₀), preferably (C₁₄₋C₁₈), alkyl chain, such as, for instance, N-stearylamine, N,N'-distearylamine, N-hexadecylamine, N,N'-dihexadecylamine, N-stearylammonium chloride, N,N'-distearylammonium chloride, N-hexadecylammonium chloride, N,N'-dihexadecylammonium chloride, dimethyldioctadecylammonium bromide (DDAB), hexadecyltrimethylammonium bromide (CTAB); tertiary or quaternary ammonium salts comprising one or preferably two (C₁₀-C₂₀), preferably (C₁₄-C₁₈), acyl chain linked to the N-atom through a (C₃-C₆) alkylene bridge, such as, for instance, 1,2-distearoyl-3-trimethylammonium-propane (DSTAP), 1,2-dipalmitoyl-3-trimethylammonium-propane (DPTAP), 1,2-oleoyl-3-trimethylammonium-propane (DOTAP), 1,2-distearoyl-3-dimethylammonium-propane (DSDAP); and mixtures or combinations thereof.

Depending on the combination of components and on the manufacturing process of the microbubbles, the above listed exemplary compounds may be employed as main compound for forming the microbubble's envelope or as simple additives, thus being present only in minor amounts.

According to a preferred embodiment, at least one of the compounds forming the microbubbles' envelope is a phospholipid, optionally in admixture with any of the other above cited film-forming materials. According to the present description, the term phospholipid is intended to encompass any amphiphilic phospholipid compound, the molecules of which are capable of forming a stabilizing film of material (typically in the form of a mono-molecular layer) at the gas-water boundary interface in the final microbubbles suspension. Accordingly, these materials are also referred to in the art as "film-forming phospholipids".

Amphiphilic phospholipid compounds typically contain at least one phosphate group and at least one, preferably two, lipophilic long-chain hydrocarbon group.

Examples of suitable phospholipids include esters of glycerol with one or preferably two (equal or different) residues of fatty acids and with phosphoric acid, wherein the phosphoric acid residue is in turn bound to a hydrophilic group, such a, for instance, choline (phosphatidylcholines - PC), serine (phosphatidylserines - PS), glycerol (phosphatidylglycerols - PG), ethanolamine (phosphatidylethanolamines - PE), inositol (phosphatidylinositol). Esters of phospholipids with only one residue of fatty acid are generally referred to in the art as the "lyso" forms of the phospholipid or "lysophospholipids". Fatty acids residues present in the phospholipids are in general long chain aliphatic acids, typically containing from 12 to 24 carbon atoms, preferably from 14 to 22; the aliphatic chain may contain one or more unsaturations or is preferably completely saturated. Examples of suitable fatty acids included in the phospholipids are, for instance, lauric acid, myristic acid, palmitic acid, stearic acid, arachidic acid, behenic acid, oleic acid, linoleic acid, and linolenic acid. Preferably, saturated fatty acids such as myristic acid, palmitic acid, stearic acid and arachidic acid are employed.

Further examples of phospholipid are phosphatidic acids, i.e. the diesters of glycerol-phosphoric acid with fatty acids; sphingolipids such as sphingomyelins, i.e. those phosphatidylcholine analogs where the residue of glycerol diester with fatty acids is replaced by a ceramide chain; cardiolipins, i.e. the esters of 1,3-diphosphatidylglycerol with a fatty acid; glycolipids such as gangliosides GM1 (or GM2) or cerebrosides; glucolipids; sulfatides and glycosphingolipids.

As used herein, the term phospholipids include either naturally occurring, semisynthetic or synthetically prepared products that can be employed either singularly or as mixtures. Examples of naturally occurring phospholipids are natural lecithins (phosphatidylcholine (PC) derivatives) such as, typically, soya bean or egg yolk lecithins.

Examples of semisynthetic phospholipids are the partially or fully hydrogenated derivatives of the naturally occurring lecithins. Preferred phospholipids are fatty acids di-esters of phosphatidylcholine, ethylphosphatidylcholine, phosphatidylglycerol, phosphatidic acid, phosphatidylethanolamine, phosphatidylserine, phosphatidylinositol or of sphingomyelin.

Examples of preferred phospholipids are, for instance, dilauroyl-phosphatidylcholine (DLPC), dimyristoyl-phosphatidylcholine (DMPC), dipalmitoyl-phosphatidylcholine (DPPC), diarachidoyl-phosphatidylcholine (DAPC), distearoyl-phosphatidylcholine (DSPC), dioleoyl-phosphatidylcholine (DOPC), 1,2 Distearoyl-sn-glycero-3-Ethylphosphocholine (Ethyl-DSPC), dipentadecanoyl-phosphatidylcholine (DPDPC), 1-myristoyl-2-palmitoyl-phosphatidylcholine (MPPC), 1-palmitoyl-2-myristoyl-phosphatidylcholine (PMPC), 1-palmitoyl-2-stearoyl-phosphatidylcholine (PSPC), 1-stearoyl-2-palmitoyl-phosphatidylcholine (SPPC), 1-palmitoyl-2-oleylphosphatidylcholine (POPC), 1-oleyl-2-palmitoyl-phosphatidylcholine (OPPC), dilauroyl-phosphatidylglycerol (DLPG) and its alkali metal salts, diarachidoylphosphatidyl-glycerol (DAPG) and its alkali metal salts, dimyristoylphosphatidylglycerol (DMPG) and its alkali metal salts, dipalmitoylphosphatidylglycerol (DPPG) and its alkali metal salts, distearoylphosphatidylglycerol (DSPG) and its alkali metal salts, dioleoyl-phosphatidylglycerol (DOPG) and its alkali metal salts, dimyristoyl phosphatidic acid (DMPA) and its alkali metal salts, dipalmitoyl phosphatidic acid (DPPA) and its alkali metal salts, distearoyl phosphatidic acid (DSPA), diarachidoylphosphatidic acid (DAPA) and its alkali metal salts, dimyristoyl-phosphatidylethanolamine (DMPE), dipalmitoylphosphatidylethanolamine (DPPE), distearoyl phosphatidyl-ethanolamine (DSPE), dioleylphosphatidyl-ethanolamine (DOPE), diarachidoylphosphatidylethanolamine (DAPE), dilinoleylphosphatidylethanolamine (DLPE), dimyristoyl phosphatidylserine (DMPS), diarachidoyl phosphatidylserine (DAPS), dipalmitoyl phosphatidylserine (DPPS), distearoylphosphatidylserine (DSPS), dioleoylphosphatidylserine (DOPS), dipalmitoyl sphingomyelin (DPSP), and distearoylsphingomyelin (DSSP), dilauroyl-phosphatidylinositol (DLPI), diarachidoylphosphatidylinositol (DAPI), dimyristoylphosphatidylinositol (DMPI), dipalmitoylphosphatidylinositol (DPPI), distearoylphosphatidylinositol (DSPI), dioleoyl-phosphatidylinositol (DOPI).

The term phospholipid further includes modified phospholipid, e.g. phospholipids where the hydrophilic group is in turn bound to another hydrophilic group. Examples of modified phospholipids are phosphatidylethanolamines modified with polyethylenglycol (PEG), i.e. phosphatidylethanolamines where the hydrophilic ethanolamine moiety is linked to a PEG molecule of variable molecular weight (e.g. from 300 to 5000 daltons), such as DPPE-PEG (or DSPE-, DMPE- or DAPE-PEG), i.e. DPPE (or DSPE, DMPE, or DAPE) having a PEG polymer attached thereto. For example, DPPE-PEG2000 refers to DPPE having attached thereto a PEG polymer having a mean average molecular weight of about 2000.

Particularly preferred phospholipids are DAPC, DSPC, DPPA, DSPA, DMPS, DPPS, DSPS and Ethyl-DSPC. Most preferred are DPPS or DSPC.

Mixtures of phospholipids can also be used, such as, for instance, mixtures of DPPE, DPPC, DSPC and/or DAPC with DSPS, DPPS, DSPA, DPPA, DSPG, DPPG, Ethyl-DSPC and/or Ethyl-DPPC.

In preferred embodiments, the phospholipid is the main component of the stabilizing envelope of microbubbles, amounting to at least 50% (w/w) of the total amount of components forming the envelope of the gas filled microbubbles. In some of the preferred embodiments, substantially the totality of the envelope (i.e. at least 90% and up to 100% by weight) can be formed of phospholipids.

The phospholipids can conveniently be used in admixture with any of the above listed amphiphilic compounds. Thus, for instance, lipids such as cholesterol, ergosterol, phytosterol, sitosterol, lanosterol, tocopherol, propyl gallate or ascorbyl palmitate, fatty acids such as myristic acid, palmitic acid, stearic acid, arachidic acid and derivatives thereof or butylated hydroxytoluene and/or other non-phospholipid compounds can optionally be added to one or more of the foregoing phospholipids in proportions ranging from zero to 50% by weight, preferably up to 25%. Particularly preferred is palmitic acid.

According to a preferred embodiment, the envelope of microbubbles includes a compound bearing an overall (positive or negative) net charge. Said compound can be a charged amphiphilic material, preferably a lipid or a phospholipid.

Examples of phospholipids bearing an overall negative charge are derivatives, in particular fatty acid di-ester derivatives, of phosphatidylserine, such as DMPS, DPPS, DSPS; of phosphatidic acid, such as DMPA, DPPA, DSPA; of phosphatidylglycerol such as DMPG, DPPG and DSPG or of phosphatidylinositol, such as DMPI, DPPI or DPPI. Also modified phospholipids, in particular PEG-modified phosphatidylethanolamines, such as DMPE-PEG1000, DMPE-PEG2000, DMPE-PEG3000, DMPE-PEG4000, DMPE-PEG5000, DPPE-PEG1000, DPPE-PEG2000, DPPE-PEG3000, DPPE-PEG4000, DPPE-PEG5000, DSPE-PEG1000, DSPE-PEG2000, DSPE-PEG3000, DSPE-PEG4000, DSPE-PEG5000, DAPE-PEG1000, DAPE-PEG2000, DAPE-PEG3000, DAPE-PEG4000 or DAPE-PEGS000 can be used as negatively charged molecules. Also the lyso- form of the above cited phospholipids, such as lysophosphatidylserine derivatives (e.g. lyso-DMPS, -DPPS or -DSPS), lysophosphatidic acid derivatives (e.g. lyso-DMPA, -DPPA or -DSPA) and lysophosphatidylglycerol derivatives (e.g. lyso-DMPG, -DPPG or -DSPG), can advantageously be used as negatively charged compound. Examples of negatively charged lipids are bile acid salts such as cholic acid salts, deoxycholic acid salts or glycocholic acid salts; and (C₁₂-C₂₄), preferably (C₁₄-C₂₂) fatty acid salts such as, for instance, palmitic acid salt, stearic acid salt, 1,2-dipalmitoyl-sn-3-succinylglycerol salt or 1,3-dipalmitoyl-2-succinylglycerol salt.

Preferably, the negatively charged compound is selected among DPPA, DPPS, DSPG, DSPE-PEG2000, DSPE-PEG5000 or mixtures thereof.

The negatively charged component is typically associated with a corresponding positive counter-ion, which can be mono- (e.g. an alkali metal or ammonium), di- (e.g. an earth-alkali metal) or tri-valent (e.g. aluminium). Preferably the counter-ion is selected among alkali metal cations, such as Li⁺, Na⁺, or K⁺, more preferably Na⁺.

Examples of phospholipids bearing an overall positive charge are derivatives of ethylphosphatidylcholine, in particular di-esters of ethylphosphatidylcholine with fatty acids, such as 1,2-Distearoyl-sn-glycero-3-Ethylphosphocholine (Ethyl-DSPC or DSEPC), 1,2-Dipalmitoyl-sn-glycero-3-Ethylphosphocholine (Ethyl-DPPC or DPEPC). The negative counterion is preferably an halogen ion, in particular chlorine or bromine. Examples of positively charged lipids are alkylammonium salts with a halogen counter ion (e.g. chlorine or bromine) comprising at least one (C₁₀-C₂₀), preferably (C₁₄-C₁₈), alkyl chain, such as, for instance mono or di-stearylammonium chloride, mono or di-hexadecylammonium chloride, dimethyldioctadecylammonium bromide (DDAB), hexadecyltrimethylammonium bromide (CTAB). Further examples of positively charged lipids are tertiary or quaternary ammonium salts with a halogen counter ion (e.g. chlorine or bromine) comprising one or preferably two (C₁₀-C₂₀), preferably (C₁₄₋C₁₈), acyl chain linked to the N-atom through a (C₃-C₆) alkylene bridge, such as, for instance, 1,2-distearoyl-3-trimethylammonium-propane (DSTAP), 1,2-dipalmitoyl-3-trimethylammonium-propane (DPTAP), 1,2-oleoyl-3-trimethylammonium-propane (DOTAP), 1,2-distearoyl-3-dimethylammonium-propane (DSDAP).

DSEPC, DPEPC and/or DSTAP are preferably employed as positively charged compounds in the microbubbles envelope.

The positively charged component is typically associated with a corresponding negative counter-ion, which can be mono- (e.g. halogen), di- (e.g. sulphate) or tri-valent (e.g. phosphate). Preferably the counter-ion is selected among halogen ions, such as F-(fluorine), Cl- (chlorine) or Br⁻ (bromine).

Mixtures of neutral and charged compounds, in particular of phospholipids and/or lipids, can be satisfactorily employed to form the microbubbles envelope. The amount of charged lipid or phospholipid may vary from about 95% to about 1% by mole, with respect to the total amount of lipid and phospholipid, preferably from 80% to 20% by mole.

Preferred mixtures of neutral phospholipids and charged lipids or phospholipids are, for instance, DPPG/DSPC , DSTAP/DAPC, DPPS/DSPC, DPPS/DAPC, DPPE/DPPG, DSPA/DAPC, DSPA/DSPC and DSPG/DSPC.

Other excipients or additives may be present either in the dry formulation of the microbubbles or may be added together with the aqueous carrier used for the reconstitution thereof, without necessarily being involved (or only partially involved) in the formation of the stabilizing envelope of the microbubble. These include pH regulators, osmolality adjusters, viscosity enhancers, emulsifiers, bulking agents, etc. and may be used in conventional amounts. For instance compounds like polyoxypropylene glycol and polyoxyethylene glycol as well as copolymers thereof can be used. Examples of viscosity enhancers or stabilizers are compounds selected from linear and cross-linked poly- and oligo-saccharides, sugars, hydrophilic polymers like polyethylene glycol.

As the preparation of gas-filled microbubbles may involve a freeze drying or spray drying step, it may be advantageous to include in the formulation a lyophilization additive, such as an agent with cryoprotective and/or lyoprotective effect and/or a bulking agent, for example an amino-acid such as glycine; a carbohydrate, e.g. a sugar such as sucrose, mannitol, maltose, trehalose, glucose, lactose or a cyclodextrin, or a polysaccharide such as dextran; or a polyglycol such as polyethylene glycol.

The microbubbles can be produced according to any known method in the art. Typically, the manufacturing method involves the preparation of a dried powdered material comprising an amphiphilic material as above indicated, preferably by lyophilization (freeze drying) of an aqueous or organic suspension comprising said material.

For instance, as described in WO 91/15244 film-forming amphiphilic compounds can be first converted into a lamellar form by any liposome forming method. To this end, an aqueous solution comprising the film forming lipids and optionally other additives (e.g. viscosity enhancers, non-film forming surfactants, electrolytes etc.) can be submitted to high-speed mechanical homogenisation or to sonication under acoustical or ultrasonic frequencies, and then freeze dried to form a free flowable powder which is then stored in the presence of a gas. Optional washing steps, as disclosed for instance in US 5,597,549, can be performed before freeze drying.

According to an alternative embodiment (described for instance in US 5,597,549) a film forming compound and a hydrophilic stabiliser (e.g. polyethylene glycol, polyvinyl pyrrolidone, polyvinyl alcohol, glycolic acid, malic acid or maltol) can be dissolved in an organic solvent (e.g. tertiary butanol, 2-methyl-2-butanol or C₂Cl₄F₂) and the solution can be freeze-dried to form a dry powder.

Preferably, as disclosed in WO 04/069284, a phospholipid (selected among those cited above and including at least one of the above-identified charged phospholipids) and a lyoprotecting agent (such as those previously listed, in particular carbohydrates, sugar alcohols, polyglycols and mixtures thereof) can be dispersed in an emulsion of water with a water immiscible organic solvent (e.g. branched or linear alkanes, alkenes, cycloalkanes, aromatic hydrocarbons, alkyl ethers, ketones, halogenated hydrocarbons, perfluorinated hydrocarbons or mixtures thereof) under agitation. The emulsion can be obtained by submitting the aqueous medium and the solvent in the presence of at least one phospholipid to any appropriate emulsion-generating technique known in the art, such as, for instance, sonication, shaking, high pressure homogenization, micromixing, membrane emulsification, high speed stirring or high shear mixing. For instance, a rotor-stator homogenizer can be employed, such as Polytrono® PT3000. The agitation speed of the rotor-stator homogenizer can be selected depending from the components of the emulsion, the volume of the emulsion, the relative volume of organic solvent, the diameter of the vessel containing the emulsion and the desired final diameter of the microdroplets of solvent in the emulsion. Alternatively, a micromixing technique can be employed for emulsifying the mixture, e.g. by introducing the organic solvent into the mixer through a first inlet (at a flow rate of e.g. 0.05-5 ml/min), and the aqueous phase a second inlet (e.g. at a flow rate of 2-100 ml/min). The outlet of the micromixer is then connected to the vessel containing the aqueous phase, so that the aqueous phase drawn from said vessel at subsequent instants and introduced into the micromixer contains increasing amounts of emulsified solvent. When the whole volume of solvent has been added, the emulsion from the container can be kept under recirculation through the micromixer for a further predetermined period of time, e.g. 5-120 minutes, to allow completion of the emulsion. Depending on the emulsion technique, the organic solvent can be introduced gradually during the emulsification step or at once before starting the emulsification step. Alternatively the aqueous medium can be gradually added to the water immiscible solvent during the emulsification step or at once before starting the emulsification step. Preferably, the phospholipid is dispersed in the aqueous medium before this latter is admixed with the organic solvent. Alternatively, the phospholipid can be dispersed in the organic solvent or it may be separately added the aqueous-organic mixture before or during the emulsification step. The so obtained microemulsion, which contains microdroplets of solvent surrounded and stabilized by the phospholipid material (and optionally by other amphiphilic film-forming compounds and/or additives), is then lyophilized according to conventional techniques to obtain a lyophilized material, which is stored (e.g. in a vial in the presence of a suitable gas) and which can be reconstituted with an aqueous carrier to finally give a gas-filled microbubbles suspension where the dimensions and size distribution of the microbubbles are substantially comparable with the dimensions and size distribution of the suspension of microdroplets.

A further process for preparing gas-filled microbubbles comprises generating a gas microbubble dispersion by submitting an aqueous medium comprising a phospholipid (and optionally other amphiphilic film-forming compounds and/or additives) to a controlled high agitation energy (e.g. by means of a rotor stator mixer) in the presence of a desired gas and subjecting the obtained dispersion to lyophilisation to yield a dried reconstitutable product. An example of this process is given, for instance, in WO 97/29782, here enclosed by reference.

Spray drying techniques (as disclosed for instance in US 5,605,673) can also be used to obtain a dried powder, reconstitutable upon contact with physiological aqueous carrier to obtain gas-filled microbubbles.

The dried or lyophilised product obtained with any of the above techniques will generally be in the form of a powder or a cake, and can be stored (e.g. in a vial) in contact with the desired gas. The product is readily reconstitutable in a suitable physiologically acceptable aqueous liquid carrier, which is typically injectable, to form the gas-filled microbubbles, upon gentle agitation of the suspension. Suitable physiologically acceptable liquid carriers are sterile water, aqueous solutions such as saline (which may advantageously be balanced so that the final product for injection is not hypotonic), or solutions of one or more tonicity adjusting substances such as salts or sugars, sugar alcohols, glycols or other non-ionic polyol materials (eg. glucose, sucrose, sorbitol, mannitol, glycerol, polyethylene glycols, propylene glycols and the like).

Mean dimensions and size distribution of the final reconstituted microbubbles can be in general be determined by suitably acting on the parameters of the preparation process. In general, different values of mean size and size distribution of a final preparation can be obtained by selecting different envelope-stabilizing phospholipids and/or (when required by the process) by the selection of different organic solvents and/or different volumes thereof (relative to the volume of aqueous phase). In addition, for the specific preparation processes disclosed in WO 04/069284 or WO97/29782, a variation of the mixing speed generally results in a variation of the mean dimensions of the final microbubble preparation (typically, the higher the mixing speeds, the smaller the obtained microbubbles).

According to a preferred alternative embodiment, the composition of the invention may comprise any suitable ultrasound contrast agent in the form of microballons.

As formerly reported, gas-filled microballoons as defined herein comprise microvesicles having a material envelope, the thickness of which is in general much greater than the thickness of microbubbles stabilizing film-envelope. Depending from the material forming said envelope (which can be e.g. polymeric, proteinaceous, of a water insoluble lipid or of any combination thereof), said thickness is in general of at least 50 nm, typically of at least 100 nm, up to few hundred nanometers (e.g. 300 nm).

Microballoons also generally differ from microbubbles in terms of acoustic response to ultrasonication. While the ultrasonic behavior of microbubbles is in fact closer to the behavior of "free" gas bubbles, microballoons (probably because of a higher stiffness of the envelope) are in general less responsive (in terms of intensity of the reflected echo signal) when irradiated at low levels of acoustic pressure energy (e.g. at a mechanical index of about 0.1).Microballoons

Preferred examples of microballoons are those comprising a stabilizing polymeric envelope, preferably comprising a biodegradable polymer, or a stabilizing envelope based on biodegradable water-insoluble lipids, such as, for instance those described in US 5,711,933 and US 6,333,021, herein incorporated by reference in their entirety. Microballoons having a proteinaceous envelope, i.e. made of natural proteins (albumin, haemoglobin) such as those described in US-A-4,276,885 or EP-A-0 324 938, can also be employed. Polymers forming the envelope of the injectable microballoons are preferably hydrophilic, biodegradable physiologically compatible polymers. Examples of such polymers, which may be natural or synthetic, are substantially insoluble polysaccharides (e.g. chitosan or chitin), polycyanoacrylates, polylactides and polyglycolides and their copolymers, copolymers of lactides and lactones such as γ-caprolactone or δ-valerolactone, copolymers of ethyleneoxide and lactides, polyethyleneimines, polypeptides, and proteins such as gelatin, collagen, globulins or albumins. Other suitable polymers mentioned in the above cited US 5,711,933 include poly-(ortho)esters, polylactic and polyglycolic acid and their copolymers (e.g. DEXON®, Davis & Geck, Montreal, Canada); poly(DL-lactide-co-γ-caprolactone), poly(DL-lactide-co-δ-valerolactone), poly(DL-lactide-co-γ-butyrolactone), polyalkylcyanoacrylates; polyamides, polyhydroxybutyrate; polydioxanone; poly-β-aminoketones; polyphosphazenes; and polyanhydrides. Polyamino-acids such as polyglutamic and polyaspartic acids can also be used, as well as their derivatives, such as partial esters with lower alcohols or glycols. Copolymers with other amino acids such as methionine, leucine, valine, proline, glycine, alanine, etc. can also be used. Derivatives of polyglutamic and polyaspartic acid with controlled biodegradability (such as those described in WO87/03891, US 4,888,398 or EP 130935, all herein incorporated by reference) can also be used. These polymers (and copolymers with other amino-acids) have formulae of the following type: -(NH-CHA-CO)_{w} -(NH-CHX-CO)y- where X designates the side chain of an amino acid residue (e.g. methyl, isopropyl, isobutyl, or benzyl); A is a group of formula -(CH₂)ₙ COOR¹ R² -OCOR, -(CH₂)ₙ COO-CHR¹COOR, -(CH₂)ₙ CO(NH-CHX-CO)ₘ NH-CH(COOH)-(CH₂)ₚ COOH, or the respective anhydrides thereof, wherein R¹ and R² represent H or lower alkyls, and R represents alkyl or aryl; or R and R¹ are connected together by a substituted or unsubstituted linking member to provide 5- or 6- membered rings; n, m and p are lower integers, not exceeding 5; and w and y are integers selected for having molecular weights not below 5000. Non-biodegradable polymers (e.g. for making microballoons to be used in the digestive tract) can be selected from most water-insoluble, physiologically acceptable, bioresistant polymers including polyolefins (polystyrene), acrylic resins (polyacrylates, polyacrylonitrile), polyesters (polycarbonate), polyurethanes, polyurea and their copolymers. ABS (acryl-butadiene-styrene) is a preferred copolymer.

Biodegradable water-insoluble lipids useful for forming a microballoon comprise, for instance, solid water insoluble mono-, di- or tri-glycerides, fatty acids, fatty acid esters, sterols such as cholesterol, waxes and mixtures thereof. Mono-, di- and tri- glycerides include mainly the mono-, di- and tri-laurin compounds as well as the corresponding - myristin, -palmitin, -stearin, -arachidin and -behenin derivatives. Mono-, di- and triarachidin, -palmitin -stearin and mixed triglycerides such as dipalmitoylmonooleyl glyceride are particularly useful; tripalmitin and tristearin are preferred. Fatty acids include solid (at room temperature, about 18-25°C) fatty acids (preferably saturated) having 12 carbon atoms or more, including, for instance, lauric, arachidic, behenic, palmitic, stearic, sebacic, myristic, cerotinic, melissic and erucic acids and the fatty acid esters thereof. Preferably, the fatty acids and their esters are used in admixture with other glycerides.

The sterols are preferably used in admixture with the other glycerides and or fatty acids and are selected from cholesterol, phytosterol, lanosterol, ergosterol, etc. and esters of the sterols with the above mentioned fatty acids; however, cholesterol is preferred.

Preferred biodegradable lipids are triglycerides such as tripalmitin, triarachidin, tristearin or mixtures of the above mentioned triglycerides.

Optionally, up to 75% by weight of a biodegradable polymer, such as those listed previously, can be admixed together with the biodegradable water insoluble lipid forming the envelope of the microballoon.

Advantageously, ionic polymers (i.e. polymers bearing ionic moieties in their structure), preferably biodegradable ionic polymers, can also be used to form the stabilizing envelope of the microballoons, thus conferring the desired overall net charge thereto. Ionic polymers can be used as main components of the stabilizing envelope or they can be admixed in various amounts (e.g. from 2 to 80% by weight) with non ionic polymers. Suitable ionic polymers are, for instance, polymers comprising a quaternized nitrogen atom, such as quaternized amines or polymers comprising an carboxylic, sulfate, sulfonate or phosphonate moieities. Examples of suitable ionic polymers include, without limitation, polyethylenimine, poly(diallyldimethylammonium chloride), poly{bis(2-chloroethyl)ether-alt-1,3-bis[3-(dimethylamino)propyl]urea} quaternized (Polyquaternium®-2), poly(4-vinylpyridinium tribromide), hydroxyethylcellulose ethoxylate quaternized (Polyquaternium®-4, poly(*p*-xylene tetrahydrothiophenium chloride), poly(L-lysine), chitin, diethyleneaminoethyl dextran, poly(acrylic acid), poly(methacrylic acid), poly(styrene-*alt*-maleic acid), poly(amino acids), alginic acid, poly(uridylic acid), hyaluronic acid, i.e. poly(β-glucuronic acid-*alt*-β-N-acetylglucosamide), poly(galacturonic acid), poly(vinyl acetate-*co*-crotonic acid), DNA, poly(3,3',4,4'-benzophenonetetracarboxylic dianhydride-co-4,4'-oxydianiline), poly(isoprene-graft- maleic acid monomethyl ether), copolymer of glutamic acid with alkyl glutamate, heparin, poly(styrene sulfonate), sulfonated poly(isophthalic acid), poly(vinyl sulfonate, potassium salt), poly(vinyl sulfate, potassium salt), chondroitin sulfate A, dextran sulfate, fucoidan, polyphosphoric acid, sodium polyphosphate, sodium polyvinylphosphonate, poly-L-lysine hydrobromide, chitosan, chitosan sulfate, sodium alginate, alginic acid and ligninsulfonate.

Conventional additives can also be incorporated into the envelope of the microballoons, to modify physical properties thereof, such as dispersibility, elasticity and water permeability. In particular, effective amounts of amphiphilic materials can be added to the emulsion prepared for the manufacturing of said microballoons, in order to increase the stability thereof. Said materials can advantageously be selected among those amphiphilic compounds, such as lipids, phospholipids and modified phospholipids, listed in the foregoing of this specification.

The added amphiphilic material can advantageously be a compound bearing an overall net charge. Preferred charged lipids, phospholipids and modified phospholipids are those previously listed. Preferably the amount of charged compound, when present, is from about 2% to 40% of the total weight of the material forming the stabilizing envelope.

Other excipients or additives, in particular used for the preparation of microballoons, can be incorporated into the envelope such as redispersing agents or viscosity enhancers.

Biodegradable polymer-containing microballoons can be prepared, for instance, according to the process disclosed in US 5,711,933, herein incorporated by reference, which comprises (a) emulsifying a hydrophobic organic phase into a water phase so as to obtain droplets of said hydrophobic phase as an oil-in-water emulsion in said water phase; (b) adding to said emulsion a solution of at least one polymer in a volatile solvent insoluble in the water phase, so that said polymer forms a layer around said droplets; (c) evaporating said volatile solvent so that the polymer deposits by interfacial precipitation around the droplets which then form beads with a core of said hydrophobic phase encapsulated by a membrane of said polymer, said beads being in suspension in said water phase; (d) removing said encapsulated hydrophobic phase by evaporation by subjecting said suspension to reduced pressure; and (e) replacing said evaporated hydrophobic phase with a suitable gas.

Biodegradable lipid-containing microballoons can be prepared, for instance, according to the process disclosed in US 6,333,021 (herein incorporated by reference), by dispersing a mixture of one or more of the solid constituents of the microcapsule envelope dissolved in an organic solvent in a water carrier phase, so as to produce an oil-in-water emulsion. The emulsion water phase may contain an effective amount of amphiphilic materials which are used to stabilise the emulsion.

A certain amount of redispersing agent and/or of a cryoprotecting or lyoprotecting agent, such as those previously indicated, is then added to the emulsion of tiny droplets of the organic solution in the water phase, prior to freezing at a temperature below -30°C. Any convenient redispersing agent may be used; redispersing agents selected from sugars, albumin, gelatine, polyvinyl pyrolidone (PVP), polyvinyl alcohol (PVA), polyethylene glycol (PEG) and ethyleneoxide-propyleneoxide block copolymer (e.g. Pluronic®, or Synperonic®) or mixtures thereof are preferred. The redispersing agents which are added to prevent particle agglomeration are particularly useful when the microcapsules are in the form of non-coalescent, dry and instantly dispersible powders. The frozen emulsion is then subjected to reduced pressure to effect lyophilisation, i.e. the removal by sublimation of the organic solvent from the droplets and of the water of the carrier phase, and the freeze-dried product is then contacted with the desired gas.

The microballoons can then be reconstituted by contacting the dried powder with a suitable aqueous carrier under gentle agitation.

As far as the gas filling in the above microbubbles or microballoons is concerned, any biocompatible gas, gas precursor or mixture thereof may be employed for the preparation of the above microvesicles.

The gas may comprise, for example, air; nitrogen; oxygen; carbon dioxide; hydrogen; nitrous oxide; a noble or inert gas such as helium, argon, xenon or krypton; a radioactive gas such as Xe¹³³ or Kr⁸¹; a hyperpolarized noble gas such as hyperpolarized helium, hyperpolarized xenon or hyperpolarized neon; a low molecular weight hydrocarbon (e.g. containing up to 7 carbon atoms), for example an alkane such as methane, ethane, propane, butane, isobutane, pentane or isopentane, a cycloalkane such as cyclobutane or cyclopentane, an alkene such as propene, butene or isobutene, or an alkyne such as acetylene; an ether; a ketone; an ester; halogenated gases, preferably fluorinated gases, such as or halogenated, fluorinated or prefluorinated low molecular weight hydrocarbons (e.g. containing up to 7 carbon atoms); or a mixture of any of the foregoing. Where a halogenated hydrocarbon is used, preferably at least some, more preferably all, of the halogen atoms in said compound are fluorine atoms.

Fluorinated gases are preferred, in particular perfluorinated gases, especially in the field of ultrasound imaging. Fluorinated gases include materials which contain at least one fluorine atom such as, for instance fluorinated hydrocarbons (organic compounds containing one or more carbon atoms and fluorine); sulfur hexafluoride; fluorinated, preferably perfluorinated, ketones such as perfluoroacetone; and fluorinated, preferably perfluorinated, ethers such as perfluorodiethyl ether. Preferred compounds are perfluorinated gases, such as SF₆ or perfluorocarbons (perfluorinated hydrocarbons), i.e. hydrocarbons where all the hydrogen atoms are replaced by fluorine atoms, which are known to form particularly stable microbubble suspensions, as disclosed, for instance, in EP 0554 213, which is herein incorporated by reference.

The term perfluorocarbon includes saturated, unsaturated, and cyclic perfluorocarbons. Examples of biocompatible, physiologically acceptable perfluorocarbons are: perfluoroalkanes, such as perfluoromethane, perfluoroethane, perfluoropropanes, perfluorobutanes (e.g. perfluoro-n-butane, optionally in admixture with other isomers such as perfluoro-isobutane), perfluoropentanes, perfluorohexanes or perfluoroheptanes; perfluoroalkenes, such as perfluoropropene, perfluorobutenes (e.g. perfluorobut-2ene) or perfluorobutadiene; perfluoroalkynes (e.g. perfluorobut-2-yne); and perfluorocycloalkanes (e.g. perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane and perfluorocycloheptane). Preferred saturated perfluorocarbons have the formula CₙFₙ₊₂, where n is from 1 to 12, preferably from 2 to 10, most preferably from 3 to 8 and even more preferably from 3 to 6. Suitable perfluorocarbons include, for example, CF₄, C₂F₆, C₃F₈, C₄F₈, C₄F₁₀, C₅F₁₂, C₆F₁₂, C₆F₁₄, C₇F₁₄, C₇F₁₆, C₈F₁₈, and C₉F₂₀.

Particularly preferred gases are SF₆ or perfluorocarbons selected from CF₄, C₂F₆, C₃F₈, C₄F₈, C₄F₁₀ or mixtures thereof; SF₆, C₃F₈ or C₄F₁₀ are particularly preferred.

It may also be advantageous to use a mixture of any of the above gases in any ratio. For instance, the mixture may comprise a conventional gas, such as nitrogen, air or carbon dioxide and a gas forming a stable microbubble suspension, such as sulfur hexafluoride or a perfluorocarbon as indicated above. Examples of suitable gas mixtures can be found, for instance, in WO 94/09829, which is herein incorporated by reference. The following combinations are particularly preferred: a mixture of gases (A) and (B) in which the gas (B) is a fluorinated gas, preferably selected from SF₆, CF₄, C₂F₆, C₃F₆, C₃F₈, C₄F₆, C₄F₈, C₄F₁₀, C₅F₁₀, C₅F₁₂ or mixtures thereof, and (A) is selected from air, oxygen, nitrogen, carbon dioxide or mixtures thereof. The amount of gas (B) can represent from about 0.5% to about 95% v/v of the total mixture, preferably from about 5% to 80%.

In certain circumstances it may be desirable to include a precursor to a gaseous substance (i.e. a material that is capable of being converted to a gas in vivo). Preferably the gaseous precursor and the gas derived therefrom are physiologically acceptable. The gaseous precursor may be pH-activated, photo-activated, temperature activated, etc. For example, certain perfluorocarbons may be used as temperature activated gaseous precursors. These perfluorocarbons, such as perfluoropentane or perfluorohexane, have a liquid/gas phase transition temperature above room temperature (or the temperature at which the agents are produced and/or stored) but below body temperature; thus, they undergo a liquid/gas phase transition and are converted to a gas within the human body.

For ultrasonic echography, the biocompatible gas or gas mixture is preferably selected from air, nitrogen, carbon dioxide, helium, krypton, xenon, argon, methane, halogenated hydrocarbons (including fluorinated gases such as perfluorocarbons and sulfur hexafluoride) or mixtures thereof. Advantageously, perfluorocarbons (in particular C₄F₁₀ or C₃F₈) or SF₆ can be used, optionally in admixture with air or nitrogen.

For the use in MRI the microvesicles will preferably contain a hyperpolarized noble gas such as hyperpolarized neon, hyperpolarized helium, hyperpolarized xenon, or mixtures thereof, optionally in admixture with air, CO₂, oxygen, nitrogen, helium, xenon, or any of the halogenated hydrocarbons as defined above.

For use in scintigraphy, the microvesicle will preferably contain radioactive gases such as Xe¹³³ or Kr⁸¹ or mixtures thereof, optionally in admixture with air, CO₂, oxygen, nitrogen, helium, kripton or any of the halogenated hydrocarbons as defined above.

Going back to the compositions of the present invention and according to an additional preferred embodiment, the suspension of gas-containing microvesicles are selected from aqueous suspensions of amphiphile-stabilized microbubbles or microballoons.

More preferably, the said suspended microvesicles are characterized by a gaseous core and a stabilizing coat wherein said coat comprises a layer or multiple layers of self-assembling amphiphilic compounds or a solid layer of elastic polymeric material or a solid or liquid layer of a fat.

Preferably, the stabilizing coat comprises: phospholipids; phosphatidic acids; biocompatible and biodegradable polymers; triglycerides; detergents, preferably fatty acids; or any mixture thereof.

According to another embodiment of the invention, the gas of the gas-containing microvesicles is selected from nitrogen, oxygen, fluorine containing gases, noble gases and mixtures thereof.

Among the fluorine containing gases, perfluoroalkanes and sulphur hexafluoride are preferred, particularly when employed in amounts not lower than 1% (v/v) with respect to the total amount of gas.

From all of the above, non limiting examples of preferred compositions of the invention comprise ultrasound contrast agents selected from SonoVue® (Bracco), Definity® (Dupont Pharmaceutical), Imagent® (Imcor Pharmaceutical), Optison® (Amersham Health), AI700 (Acusphere) and Cardiosphere® (Point Biomedical).

According to an additional embodiment of the invention, as the targeting of cells and tissues by means of suitable ligands having a high affinity to the said cells and tissues is a rather well-known technique, for instance in the occurrence of targeted delivery of bioactive(s), the ultrasound contrast agent of the invention may also be bound to a suitable ligand which provides affinity to the cells of the lymphatic system or of the tumor.

Suitable ligands may be thus comprise, for instance, polypeptides and antibodies or their fragments and drugs, all of which having selectivity for macrophages or tumor cells. Examples of a ligands selective for certain tumor cells are: gastrin-related polypeptide, octreotide, rituxumab, infliximab, trastuzumab, adalimumab, omalizumab, tositumomab, efalizumab, cetuximab.

The contrast agent of the invention contains the echographic component at microvesicle concentrations between 10⁶/mL and 10¹⁰/mL, preferably between 10⁷/mL and 10⁹/mL. The wide concentration range is justified by the facts that: a) the echographic signal is approximatively proportional to the logarithm of the concentration; and b) reconstitution of echographic agents leads to concentrations with a substantial, but echographically irrelevant, variation.

The contrast agent of the invention contains the echographic dye at concentrations in the range from 5×10⁻⁶ M to 2 ×10⁻⁴ M, preferably in the range from 10⁻⁵ M to 5×10⁻⁵ M. The indicated wide range of concentrations reflects the fact that molar extinction coefficients of different dyes may vary greatly.

With respect to the manufacturing process, any of the aforementioned methods for preparing microbubbles and microballoons as known ultrasound contrast agents, comprehensive of any variant thereof, may apply as well to the preparation of the compositions of the invention.

Typically, the vital dye can be either present in the dried powdered material or solid or fluffy cake to be reconstituted in the form of microbubbles or microballoons before administration or, alternatively, it can be present in the solution to be used for their reconstitution.

In the former case, a suitable solution of the vital dye is properly admixed with the components of the microbubbles or microballoons themselves, for instance phospholipid components, and properly processed as above indicated, e.g. freeze-dried, so as to obtain the desired dried powdered material.

According to a preferred embodiment, however, the compositions of the invention may be prepared by reconstituting the powdered material or solid or fluffy cake with a physiological solution also comprising a proper amount of the vital dye.

Therefore, it represents a further object of the invention a process for manufacturing the compositions of the invention which process comprises reconstituting a dried powdered material or solid or fluffy cake, properly stored in the presence of a suitable microvesicle-forming gas, with a suitable aqueous carrier for injection, wherein the vital dye is present in the dried powdered material or solid or fluffy cake or in the aqueous carrier.

Preferably, as formerly indicated, the selected vital dye is present in the aqueous carrier. In the present description, unless otherwise provided, with the term solid or fluffy cake we intend the material in the state in which it is found after a lyophilization step.

Alternatively, as formerly reported, the compositions of the invention may be also prepared by converting amphiphile containing particles, being suspended in a vital dye-containing aqueous carrier for injection under a microbubble-forming gas, into a suspension of gas-containing microbubbles by vigorous agitation.

For additional details concerning the preparation processes of the compositions of the invention see, also, the subsequent experimental section.

According to the above alternative embodiments for preparing the compositions of the invention, each comprising any one of the aforementioned variants, stable formulations of ultrasound contrast agents in combination with vital dyes are thus obtained.

For an optimal visualization of the lymphatic system, the compositions of the invention may comprise microbubbles or microballons having a mean particle diameter size not exceeding about 6 µm, and preferably ranging from about 0.5 µm to about 5 µm.

Clearly, as the particles size and their frequency of distribution is known to depend on several factors, among which are the formerly reported preparation steps, possible deviations from the above mean size have to be also considered.

Even so, despite the fact that minor alteration in the chemical composition may strongly influence the yield, the distribution size and, importantly, the stability of the compositions themselves, the finding that known ultrasound contrast agents could be successfully formulated in combination with vital dyes, as per the invention, has to be certainly regarded as unexpected.

In this respect, experimental evidence is given to demonstrate the stability of some representative compositions of the invention.

More in particular, upon reconstitution of suitable freeze-dried cakes with aqueous carriers also comprising the selected vital dye, tests were performed to analyze the bubble size distribution of the microbubbles thus formed.

A combined composition according to the invention is preferably stored in dried powdered form and as such can advantageously be packaged in a two component diagnostic kit. The kit preferably comprises a first container, containing the lyophilized composition in contact with a selected microvesicle-forming gas and a second container, containing a physiologically acceptable aqueous carrier, wherein any one between the lyophilized composition or the physiologically acceptable carrier comprises the vital dye. Examples of suitable carriers are water, typically sterile, pyrogen free water (to prevent as much as possible contamination in the intermediate lyophilized product), aqueous solutions such as saline (which may advantageously be balanced so that the final product for injection is not hypotonic), or aqueous solutions of one or more tonicity adjusting substances such as salts or sugars, sugar alcohols, glycols or other non-ionic polyol materials (eg. glucose, sucrose, sorbitol, mannitol, glycerol, polyethylene glycols, propylene glycols and the like).

Preferably, the said physiologically acceptable water carrier also comprises the vital dye.

Said two component kit can include two separate containers or a dual-chamber container. In the former case the container is preferably a conventional septum-sealed vial, wherein the vial containing the lyophilized residue is sealed with a septum through which the carrier liquid may be injected using an optionally prefilled syringe. In such a case the syringe used as the container of the second component may be also then used for injecting the composition of the invention. In the latter case, the dual-chamber container is preferably a dual-chamber syringe and once the lyophilisate has been reconstituted and then suitably mixed or gently shaken, the container can be used directly for injecting the composition.

The compositions of the present invention may be used in a variety of diagnostic ultrasound imaging techniques comprising, for instance, fundamental and harmonic B-mode imaging, pulse or phase inversion imaging and fundamental and harmonic Doppler imaging, imaging of ultrasound-provoked contrast agent rupture; if desired three-dimensional imaging techniques may be used.

As the compositions of the invention and, in particular, the gas-filled microvesicles, upon injection remain intact in sufficient proportion, both the microvesicles themselves as well as the vital dye are taken up by the lymphatic system and retained in the sentinel lymph node.

Taking advantage of it, the surgeon may perform a very sensitive and effective ultrasound detection of the sentinel lymph node and thus identify the optimal site of operative incision just prior to surgery, whilst allowing optical visualization of the color marked sentinel lymph node or nodes of the tumor and optionally providing for its excision, for instance for any subsequent tissue evaluation and biopsy.

From the above, it should be clear to the skilled man that the above method allows the identification of the sentinel lymph node with a particularly non invasive technique.

In addition, the possibility of properly preparing and administering a single stable composition of the invention, also minimizes the need for subsequent administrations of the single components, for instance comprising two separated injections of the ultrasound contrast agent and of the vital dye.

It is therefore an additional object of the invention a method for the identification of the sentinel lymph node or nodes of a tumor in a mammal, which method comprises:
a) administering to said mammal a composition comprising an ultrasound contrast agent in combination with a vital dye;
b) observing by ecography the area of the lymphatic system or systems draining the tumor so as to determine the lymph node or nodes;
c) visually localizing the sentinel lymph node or nodes by surgery being guided by the coloration of the vital dye; and, optionally,
d) excising the sentinel lymph node or nodes.

Preferably, according to the method of the invention, the mammal is a human.

According to step (a), the compositions of the invention are preferably administered intradermally, subdermally, subcutaneously upstream of the lymphatic area under investigation, or specifically in the breast, also subareolarly or periareolarly.

The compositions of the invention may be typically administered at a dose corresponding to the range from about 0.001 µL to about 10 µL, preferably from about 0.01 µL to about 1 µL, of gas, depending on their respective composition, the tissue or organ to be imaged. This general dose range can of course vary further depending on specific imaging modality used, e.g. lower doses may be used when imaging with highly sensitive techniques, such as imaging based on contrast agent rupture or power pulse inversion.

In the imaging of the lymphatic system and, more particularly, of the sentinel lymph node or nodes, volumes of the compositions of the invention to be injected may preferably range from about 5 µL to about 1000 µL and, even more preferably, from about 50 µL to about 500 µL.

According to step (b) of the above method, echographic observation of the territory of interest may occur immediately after the beginning of the administration and up to a time varying from a 10 s to hours after termination of the administration, depending on the exact characteristics of the contrast composition, the administration regime and the lymphatic area under examination.

Typically, the lymph node or nodes in step (b) are those being first filled by the composition of the invention and so identified because of the echograhic contrast.

According to step (c), the ocular visualization of the lymph node or nodes occurs through a minimally invasive surgical access to the interested area, the said access being guided by the coloration of the lymphatic system brought about by the vital dye of the composition of the invention.

Once finally detected and visualized, the said lymph node or nodes may be thus subsequently analysed or anyway properly treated.

As an example, according to optional step (d), the sentinel lymph node or nodes can be surgically excised so as get histological information or, alternatively, it may be imaged in situ, for instance through magnetic resonance microscopy or single plane optical microscopy.

Alternatively, according to an additional embodiment, the compositions of the invention may be also administered as an intradermal bolus at rates preferably not exceeding 100 µL/(min and needle), followed by quantitative monitoring of the signal intensity in the lymph nodes as a function of time. The time-course may be thus used to characterize perfusion rates and sinusoidal residence times of the lymph nodes, both parameters known to be altered by the presence of obstructing tumor masses.

From all of the above, it is an additional object of the invention the use of the aforementioned compositions for the imaging of the lymphatic system and, even more particularly, for the identification of the sentinel lymph node or nodes.

With the aim of better illustrating the present invention, without posing any limitation to it, the following examples are now given.

### Example 1

A 5% (w/v) solution of Evans blue (CAS [61-73-4]), obtained from E. Merck AG, Dietikon, Switzerland, in 0.9% sodium chloride was prepared and the pH was adjusted to 7.0 with hydrochloric acid. The solution was filtered on a 0.45 µm filter. Vials of the ultrasound contrast agent, sulphur hexafluoride, phospholipids stabilized microbubbles for injection (SonoVue®, Bracco Imaging SA, Geneva, Switzerland) containing the specially formulated phospholipids cake, were reconstituted using the standard procedure, but with 5 mL of the dye solutions instead of the 0.9% sodium chloride solution. After 30 s of vigorous mixing, a dark blue suspension of microbubbles was obtained.

With the sole aim of verifying that the presence of the dye did not interfere with the known echographic contrast enhancement, once administered, the above suspension was injected intravenously at a dose of 0.03 mL/kg to a rabbit, where it provided outstanding echocardiographic views of the right and left ventricle. Even a ten fold dilution of this suspension showed strong contrast enhancement.

### Example 2

A 5% (w/v) solution of patent blue VF (CAS [129-17-9]; CI [42045]; sulphane blue), obtained from ACROS Organics Inc./Fisher Scientific, Wohlen, Switzerland, was prepared in 0.9% (w/v) sodium chloride. After complete solubilization, the pH was adjusted to 7.0 with hydrochloric acid and sodium hydroxide. The solution was filtered on a 0.22 µm single use filter.

Vials of the ultrasound contrast agent, sulphur hexafluoride, phospholipids stabilized microbubbles for injection (SonoVue®, Bracco Imaging SA, Geneva, Switzerland) containing the specially formulated phospholipids cake, were reconstituted using the standard procedure, but with 5 mL of the dye solutions instead of the 0.9% sodium chloride solution. After 30 s of vigorous mixing, a dark blue suspension of microbubbles was obtained.

### Example 3

A 8% (w/v) solution of patent blue VF (CAS [129-17-9]; CI [42045]; sulphane blue), obtained from ACROS Organics Inc./Fisher Scientific, Wohlen, Switzerland, was prepared in pharmaceutical grade water for injection, yielding an essentially isotonic solution. After complete solubilization, the pH was adjusted to 7.0 with hydrochloric acid and sodium hydroxide. The solution was filtered on a 0.22 µm single use filter.

Vials of the ultrasound contrast agent, sulphur hexafluoride, phospholipids stabilized microbubbles for injection (SonoVue®, Bracco Imaging SA, Geneva, Switzerland) containing the specially formulated phospholipids cake, were reconstituted using the standard procedure, but with 5 mL of the dye solutions instead of the 0.9% sodium chloride solution. After 30 s of vigorous mixing, a dark blue suspension of microbubbles was obtained.

### Example 4

A 4% (w/v) solution of isosulfan blue (USAN) (CAS [68238-36-8]), obtained from Sigma-Aldrich / Fluka Chemie GmbH, Buchs, Switzerland, as patent blue violet (Code # P 1888), was prepared in 0.9% (w/v) sodium chloride. After complete solubilization, the pH was adjusted to 7.0 with hydrochloric acid and sodium hydroxide. The solution was filtered on a 0.22 µm single use filter.

Vials of the ultrasound contrast agent, sulphur hexafluoride, phospholipids stabilized microbubbles for injection (SonoVue®, Bracco Imaging SA, Geneva, Switzerland) containing the specially formulated phospholipids cake, were reconstituted using the standard procedure, but with 5 mL of the dye solutions instead of the 0.9% sodium chloride solution. After 30 s of vigorous mixing, a dark blue suspension of microbubbles was obtained.

### Example 5

A 8% (w/v) solution of leuco patent blue violet (CAS [133978-89-9]), obtained from Sigma-Aldrich / Fluka Chemie GmbH, Buchs, Switzerland, Code # L 1275, was prepared in pharmaceutical grade water for injection. After complete solubilization, the pH was adjusted to 7.0 with hydrochloric acid. The solution was filtered on a 0.22 µm single use filter.

Vials of the ultrasound contrast agent, sulphur hexafluoride, phospholipids stabilized microbubbles for injection (SonoVue®, Bracco Imaging SA, Geneva, Switzerland) containing the specially formulated phospholipids cake, were reconstituted using the standard procedure, but with 5 mL of the dye solutions instead of the 0.9% sodium chloride solution. After 30 s of vigorous mixing, a dark blue suspension of microbubbles was obtained.

### Example 6

A 2.5% (w/v) solution of indocyanine green (USP) monosodium salt (CAS [3599-32-4]); sulphobromophthalein (BAN) monosodium salt; cardiogreen; foxgreen), obtained from Fluka Chemie GmbH, Buchs, Switzerland, Code # 21981, was prepared in 0.9% (w/v) sodium chloride. After complete solubilization, the pH was adjusted to 7.0. The solution was filtered on a 0.22 µm single use filter.

Vials of the ultrasound contrast agent, sulphur hexafluoride, phospholipids stabilized microbubbles for injection (SonoVue®, Bracco Imaging SA, Geneva, Switzerland) containing the specially formulated phospholipids cake, were reconstituted using the standard procedure, but with 5 mL of the dye solutions instead of the 0.9% sodium chloride solution. After 30 s of vigorous mixing, a dark green suspension of microbubbles was obtained.

### Example 7

From a 2 mL vial of perflutren lipid microbubbles (Definity®, Bristol-Myers Squibb Medical Imaging, Inc., North Billerica, MA, USA) and before its activation, 1 mL of liquid was withdrawn and replaced by a 8% (w/v) solution of isosulfan blue (USAN) (CAS [68238-36-8]). Isosulfan blue was obtained from Sigma-Aldrich / Fluka Chemie GmbH, Buchs, Switzerland, as patent blue violet (Code # P 1888) and dissolved in pharmaceutical grade water for injection. After complete solubilization, the pH was adjusted to 7.0 with hydrochloric acid and sodium hydroxide and the solution was filtered on a 0.22 µm single use filter. Upon activation of the product by agitation in the Vialmix® equipment (Bristol-Myers Squibb) the microbubble suspension formed regularly.

### Example 8

From a 3 mL vial of perflutren protein-type A microspheres for injection (USP) (Optison®, Amersham Buchler GmbH & Co. KG, Braunschweig, Germany) 1 mL of liquid was withdrawn and replaced by a 8% (w/v) solution of isosulfan blue (USAN) (CAS [68238-36-8]). Upon gentle agitation a suspension with 4.8 × 10⁸/mL microbubbles was obtained. The dye solution was prepared in the following manner: the dye was obtained from Sigma-Aldrich / Fluka Chemie GmbH, Buchs, Switzerland, as patent blue violet (Code # P 1888) and dissolved in pharmaceutical grade water for injection. After complete solubilization, the pH was adjusted to 7.0 with hydrochloric acid and sodium hydroxide and the solution was filtered on a 0.22 µm single use filter.

### Example 9

Echogenic microballoons are prepared as described in Example 3 of WO 96/15815.

Then, floating microcapsules are recovered, suspended in a 5% (w/v) mannitol solution containing Evans Blue (5% w/v) at a concentration of 5×10⁸ microballoons/mL. 1 mL of the suspension is then lyophilized. After reconstitution with 5 mL of water for injection, the resulting bubble suspension can be used for sentinel lymph node detection (see following Examples 13 and 14).

### Example 10

Dye-containing suspensions of gas-containing ultrasound contrast agents of the invention were analyzed for the particle size distributions in the Coulter® Multisizer (Beckman Coulter Inc. Fullerton, CA, USA). Depending of the preparation the suspension could contain, besides the gas-containing particles that do produce ultrasound contrast, also gas-free particles that do not. Two methods may be used to determine the distribution of just the gas-containing particles in the compositions of the invention.
Method a) The frequency distribution of the diameter of gas-containing particles is obtained as the difference between the particle size distribution before and after destruction of the gas-containing particles, in a Branson bath sonicator (Branson Ultrasonics). This method is preferable in those cases wherein, in the initial suspension, the insoluble material in the gas-containing particles is small with respect to the material in gas-free particles.
Method b) The frequency distribution of the diameter of gas-containing particles is obtained after purification of the initial particle suspension from gas-free particles. Purification is achieved by flotation of the gas-containing particles in a centrifuge and substitution of the infranatant containing the gas-free particles with fresh saline. The procedure is executed once or twice depending on the initial contamination by gas-free particles. This method is preferable in those cases wherein the amount of insoluble material in the gas-containing particles exceeds 10% of the total insoluble material, in the initial suspension.

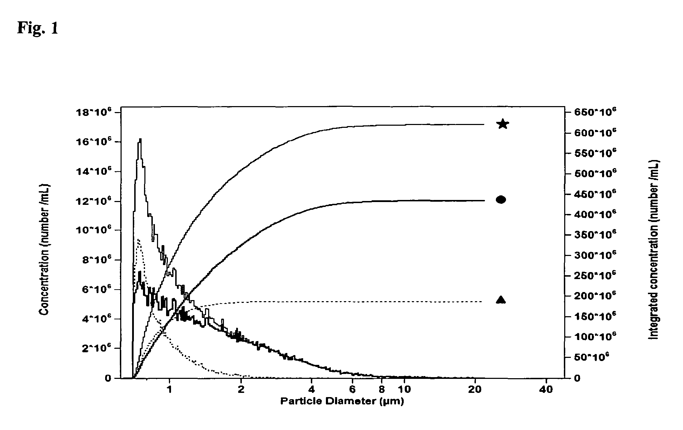

The results may be represented either in the form of plots of the frequency distributions (e.g. example 11 and Fig. 1) or as parameters elaborated therefrom, i. e. total microbubble concentration, total microbubble surface, microbubble volume concentration and mean diameter in number (e.g. example 12).

### Example 11

Dye-containing suspensions of sulphur hexafluoride, phospholipids stabilized microbubbles for injection of the invention were analyzed for the bubble size distribution according to the method (a) of Example 10.

The analysis, in particular, was carried out with the composition of the invention of example 2 (see fig. 1).
**Fig. 1**: Bubbles reconstituted with Patent blue VF (5% w/v in saline) wherein the number concentration of particles of each diameter (left scale) and the integral of the corresponding number concentration over increasing diameters (right scale) were plotted against the logarithm of the particle diameter.

The ragged curves represent particle diameter frequency distributions and the smooth curves report the corresponding integrals. The thin solid curves (★) represent the situation before destruction of the microbubbles. The dashed curves (▲) represent the situation after destruction of the microbubbles, and the thick solid curves (●) represent those that characterize the size distribution which is due to gas-filled microbubbles of the invention only.

These distributions are essentially identical to those of regularly prepared ultrasound contrast agent SonoVue®, that is, in the absence of the dye.

### Example 12

Analysis of the preparations particle size distributions in the Coulter@ Multisizer (Beckman Coulter Inc. Fullerton, CA, USA) was carried out according to example 10 (method b).

By working as therein described, the following four parameters were derived for the compositions of the invention (microbubble suspensions of example 1) in comparison with the same known microbublles (that is in the absence of the dye): total microbubble concentration, total microbubble surface, microbubble volume concentration and mean diameter in number (see Table I).

**Table I**

| **Parameters** \ **Formulation** | Formulation A | Formulation B | **Formulation C** | **Formulation D** |
|---|---|---|---|---|
| Total microbubble concentration [x 10⁸ / mL] | **3.9** | **3.3** | **2.3** | **2.0** |
| Total microbubble surface [x 10⁹ *µ*m² / mL] | **6.8** | **6.2** | **5.5** | **5.6** |
| Total microbubble volume Concentration [µL / mL] | **6.1** | **5.6** | **5.3** | **5.4** |
| Total microbubble diameter in number [µm] | **1.8** | **1.9** | **2.2** | **2.5** |

Formulation A: SonoVue reconstituted in saline, at time 0;
Formulation B: SonoVue reconstituted in saline, after 3 h;
Formulation C: SonoVue reconstituted in Evans blue, at time 0 (see example 1);
Formulation B: SonoVue reconstituted in Evans blue, after 3 h (see example 1);

In consideration of the fact that the echographic efficacy of a contrast agent is approximately proportional to the logarithm of the total microbubble concentration, comparison of SonoVue® being reconstituted with saline (hereinafter regular SonoVue®) over SonoVue® being reconstituted with saline also containing Evans blue, has unexpectedly shown that Evans blue interfered with the regular formation of microbubbles to a degree that from an echographic perspective may be considered negligible.

In addition, by comparing the above results at time 0 (that is upon microbubbles formation) and after 3 h, the presence of the Evans blue did not jeopardize the stability of the microbubbles.

### Example 13

A patient in which a breast carcinoma has been diagnosed by any combination of imaging modality and biopsy may be readied on an operating table for breast lymph node resection. Based on the imaging information the clock position (quadrant, ICD-O code) of the tumor, i.e. the direction between tumor and nipple, may be determined. Under general anesthesia an appropriate dose depending on the patient, of the blue ultrasound contrast agent of example 3 is injected intradermally, using a needle of 26 G introduced in a bevel-upward orientation at a 15° angle to the skin, stopping when the bevel dips under the skin, but the outline of the needle remains visible. The site of injection is chosen in the periareolar region in the direction of the tumor. During the whole time of injection the lymph basin draining the tumor is monitored with an ultrasound probe (15L8, Sequoia 512 from Acuson/Siemens). The first lymph node with enhanced echographic signal is identified as the sentinel lymph node. Its position is marked on the skin. About 10 min later the surgeon makes an incision and begins the search for the sentinel lymph node in the site indicated by echography, letting himself be helped further by the blue staining of the lymphatic system visible on the video screen connected to his fiberoptic telescope.

### Example 14

A patient in which a breast carcinoma has been diagnosed by any combination of imaging modality and biopsy may be readied on an operating table for breast lymph node resection. Under imaging guidance, e.g. by ultrasound imaging, the blue ultrasound contrast agent of example 3 is injected at appropriate doses into various sites immediately around the tumor. The tissue is then massaged and the sentinel lymph node is identified as the first lymph node to show increase echo. Its position is marked on the skin. About 10 min later the surgeon makes an incision and begins the search for the sentinel lymph node in the site indicated by echography, letting himself be helped further by the blue staining of the lymphatic system visible on the video screen connected to his fiberoptic telescope.

## Claims

1. A composition comprising an ultrasound contrast agent in combination with a vital dye.

2. A composition according to claim 1 wherein the ultrasound contrast agent comprises a suspension of gas-containing microvesicles.

3. A composition according to claim 2 wherein the ultrasound contrast agent is selected from SonoVue®, Definity®, Imagent®, Optison®, AI700 and Cardiosphere® (Point Biomedical).

4. A composition according to claim 3 wherein the ultrasound contrast agent is SonoVue®.

5. A composition according to claim 1 wherein the vital dye is a water soluble vital dye.

6. A composition according to claim 5 wherein the vital dye is selected from patent blue V, patent blue VF, isosulfan blue, indocyanine green monosodium salt, methylene blue, sulfobromophthaleine, copper phthalocyanine tetrasulfonate and gadolinium texaphyrin.

7. A composition according to claim 5 wherein the vital dye is selected from Evans blue and patent blue VF.

8. A process for manufacturing the compositions of claim 1 which process comprises reconstituting a dried powdered material or solid or fluffy cake, properly stored in the presence of a suitable microvesicle-forming gas, with a suitable aqueous carrier for injection, wherein the vital dye is present in the dried powdered material or solid or fluffy cake or in the aqueous carrier.

9. A process according to claim 8 wherein the vital dye is present in the aqueous carrier.

10. A kit of parts comprising a first container, containing the lyophilized composition in contact with a selected microvesicle-forming gas and a second container, containing a physiologically acceptable aqueous carrier, wherein any one between the lyophilized composition or the physiologically acceptable carrier comprises the vital dye.

11. A kit of parts according to claim 10 wherein the physiologically acceptable carrier comprises the vital dye.

12. A composition according to claim 1 for use in the preparation of a formulation for the imaging of the lymphatic system.

13. Use of the composition of claim 1 for the imaging of the lymphatic system.

14. Use of the composition of claim 1 for the identification of the sentinel lymph node or nodes of a tumor in a mammal.
